# EUROPEAN PATENT SPECIFICATION

(11) **EP 1 908 465 B1**
(45) Date of publication and mention of the grant of the patent: **29.04.2009**
(21) Application number: 06291548.3
(22) Date of filing: 04.10.2006
(51) Int. Cl.: A61K 31/155, A61P 25/00

(54) **Use of chlorine guanabenz derivatives for treating prion-based diseases**
Chlor enthaltende Guanabenz Derivate zur Behandlung von Prionerkrankungen
Utilisation de dérivées chlores du guanabenz dans le traitement des maladies à prions

(43) Date of publication of application: 09.04.2008
(73) Proprietor: CENTRE NATIONAL DE LA RECHERCHE SCIENTIFIQUE (CNRS), 75794 Paris Cedex 16 (FR); INSTITUT NATIONAL DE LA RECHERCHE AGRONOMIQUE (INRA), 75338 Paris Cédex 07 (FR)
(72) Inventor: Blondel, Marc, 29250 Saint Pol de Léon (FR); Bach, Stéphane, 29250 Saint Pol de Léon (FR); Vilette, Didier, 75013 Paris (FR); Beringue, Vincent, 91640 Janvry (FR); Tribouillard, Déborah, 29680 Roscoff (FR)
(74) Representative: Peaucelle, Chantal

(56) References cited:
- WO-A-01/00212
- WO-A-03/006426
- WO-A-20/04035815
- US-A1- 2002 150 631
- BACH S ET AL: "Isolation of drugs active against mammalian prions using a yeast-based screening assay" NATURE BIOTECHNOLOGY 01 SEP 2003 UNITED STATES, vol. 21, no. 9, 1 September 2003 (2003-09-01), pages 1075-1081, XP002426189 ISSN: 1087-0156

## Description

The invention relates to chlorine Guanabenz derivatives for treating prion-based diseases.

To date, there is no efficient treatment for prion-based diseases. Several approaches towards the development of prion disease therapies are currently explored, in particular research of pharmacological drugs promoting PrP^{sc} clearance in various cell culture assays. A few active molecules have been identified using this approach. The two most efficient were initially quinacrine (an anti-malaria agent) and chlorpromazine (an antipsychotic drug). Recently, bis-acridines, chemical dimers of quinacrine analogs tethered by a linker, have been found to be about ten fold more active than quinacrine. Because of the technical complexity inherent to these methods for example, all the manipulations have to be done in highly secure laboratories, high troughput screening has been difficult to achieve. For this reason, the inventors have developed a new, simple, economic, safe and rapid yeast-based method to screen for anti-prion drugs (Bach et al., 2003a; Bach et al., 2002; Bach et al., 2003b). In a first screen, molecules are isolated on the basis of their activity against yeast [PSI⁺] prion and then, the activity of positive compounds is confirmed against [URE3], a second yeast prion.

In a first study, the inventors screened several chemically diverse libraries of compounds (consisting of either synthetic molecules or natural products purified from various sources by academic laboratories) for the ability to cure the [*PSI⁺*] phenotype in a primary screen using a simple colorimetric reporter system. Drugs active against the [PSI⁺] phenotype were then tested for their activity against the [URE3] phenotype using a similar simple screen. Because of the structural and functional divergence of Sup35p and Ure2p proteins, molecules also active in this secondary screen were considered likely to be active against yeast prions in general. Using this two step assay, six active compounds were isolated from the first library of 2500 molecules (Bach et al., 2003a; Bach et al., 2002; Bach et al., 2003b). Five belong to a new class of molecules (kastellpaolitines), whilst the sixth is an already known molecule (phenanthridine). In addition, using a structure-activity approach, several phenanthridine derivatives were synthesized which were even more potent than the molecules identified in the screen. Very interestingly, quinacrine and chlorpromazine, the most active of the pharmacological compounds known at this date to promote mammalian prion clearance *ex vivo*, were also found to be active in the yeast-based method. Conversely all the molecules which were found positive in the yeast-based assays were efficient in promoting mammalian prion clearance in an ex *vivo* cellular system similar to the one described above (Korth et al., 2001) but also in two another mammalian cell-based assays (Archer et al., 2004; Vilette et al., 2001). Taken together, these results validate the inventors' method for finding new anti-prion drugs and furthermore suggest that, although mammalian prions and yeast prions exhibit clear differences, the biochemical pathways controlling their formation and/or maintenance are conserved from yeast to human.

Thus the present invention concerns the isolation of Guanabenz, a drug already in clinic for the treatment of hypertension, as active against prion-based diseases. Guanabenz was first isolated as active *in vivo* against yeast prions, using the two step yeast-based assay described above, and then found to be active against mammalian prion both in vitro in a cell-based assay and *in vivo* in a mouse model for prion-based disease. These results demonstrate that the treatment of prion-based diseases in mammals, and in human in particular, is a new potential therapeutic indication for Guanabenz.

By prion-based diseases, it is intended mammalian diseases due to a prion, i.e. bovine spongiform encephalopathy (mad cow disease), Creutzfeldt-Jakob disease (CJD), Gerstmann-Straussler-Scheinker syndrome, fatal familial insomia, kuru, scrapie, chronic wasting disease, feline spongiform encephalopathy and exotic ungulate encephalopathy and, preferably, bovine spongiform encephalopathy, CJD, Kuru and scrapie.

More particularly the present invention relates to the use of the molecule of formula: 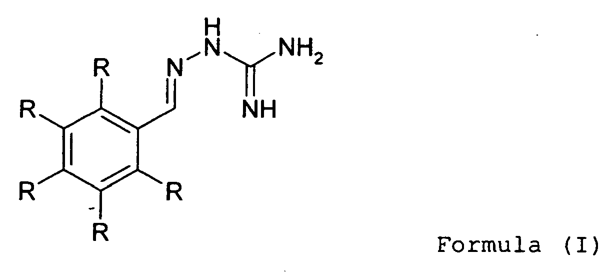 wherein R = H or Cl and the phenyl group is at least substituted twice, or a pharmaceutically acceptable salt thereof, for the manufacture of a medicament for treating prion-based diseases.

In a preferred embodiment, the molecule according to the invention is the Guanabenz, or a pharmaceutically acceptable salt thereof, for the manufacture of a medicament for treating prion-based diseases.

By the term Guanabenz, it is meant a compound of formula: 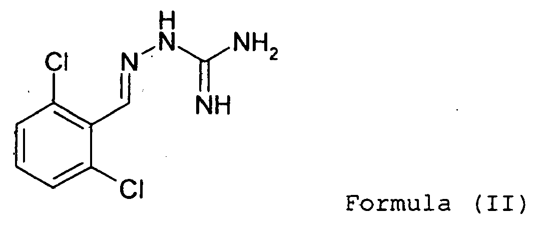 or a salt thereof, more particularly the acetate salt of formula:

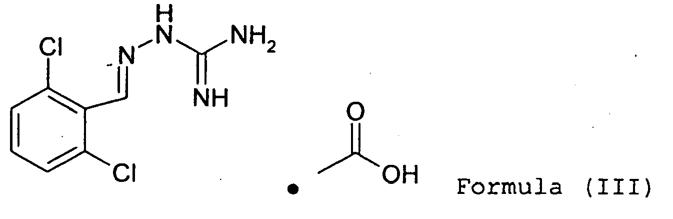

In another preferred embodiment, the molecule according to the invention is of formula: 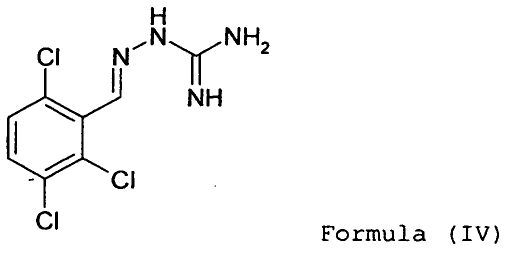 or a pharmaceutically acceptable salt thereof, for the manufacture of a medicament for treating prion-based diseases.

The present invention also relates to a method of treatment comprising the administration of a therapeutically effective amount of a compound of formula (I) to (IV) together with a pharmaceutically acceptable carrier to a patient in the need thereof.

By "treatment", it is meant reversing, alleviating, inhibiting the progress of, or preventing the disease or one or more symptoms of such disease.

By "therapeutically effective amount", it is intended an amount of a compound of the invention effective in preventing or treating pathological of prion-based diseases.

The therapeutically effective amount can be determined by the physician or anyone skilled in the art, depending of the size, age and general health of the patient, its specific disease involved and its severity, the mode of administration and other relevant circumstances. A daily dose comprises in the range of 0,01mg/kg to 0,1g/kg of body weight is preferred. However, for guanabenz acetate, the preferred daily dose range is from 0,01mg/kg to 1mg/kg of body weight, the maximum recommended human daily dose being around 1,3mg/kg.

The compounds of the invention can be delivered in different formulation, depending of the mode of administration: oral, parenteral, inhalation, topical, intracerebroventricular administration... Preferred mode of administration is oral route.

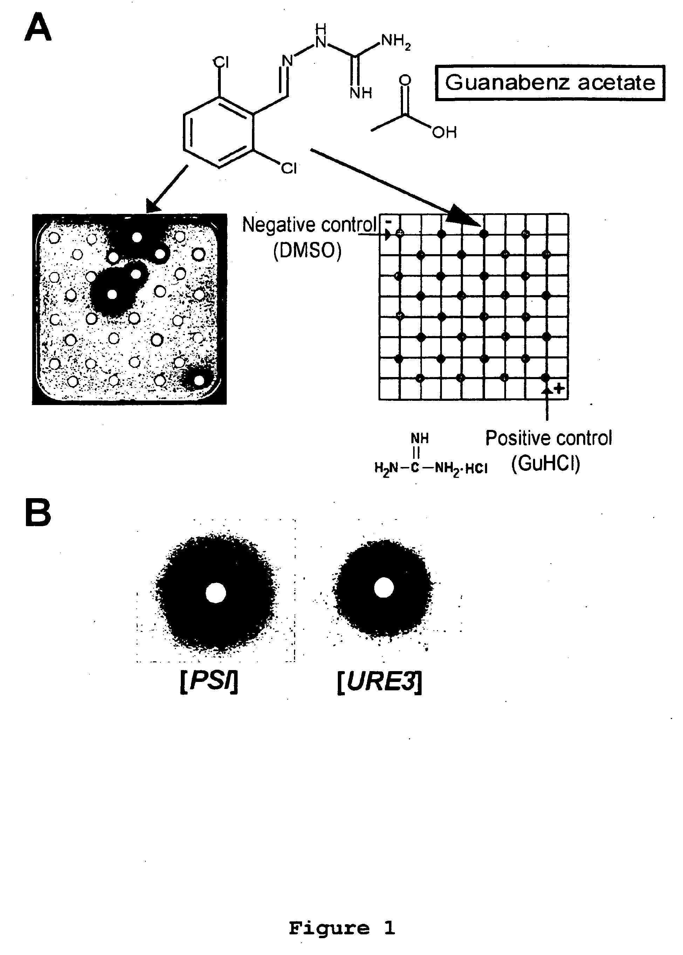

The characteristics and advantages of the present invention are illustrated by the following examples, with references to figures 1 to 4, which represent:

### Figure 1: Isolation of Guanabenz as active against yeast prion.

A. An aliquot of an overnight culture of the appropriate *erg6Δ [PSI⁺]* strain (STRg6 -which grows as white colonies-) was spread on a Petri plate containing YPD medium supplemented with 200 µM GuHCl and small filters (similar to the ones used for antibiograms) were placed on the agar surface. Individual compounds from Prestwick chemical library (5 µl of a 5 mM solution) were applied to each filter, except for the top left filter where DMSO was added (negative control: -) and for the bottom right filter where 5 µl of a 300 mM GuHCl solution in DMSO was added (positive control: +). The Petri plate was then incubated three days at 25°C. When a compound was active against *[PSI⁺],* a halo of red colonies appears around the filter where it was spotted. The molecular structure of Guanabenz acetate is depicted on top. Note that Guanabenz acetate is strongly active against *[PSI⁺]* prion.
B. Guanabenz acetate was then tested against [URE3] prion using the same kind of assay. Note that it is also active against this other yeast prion.

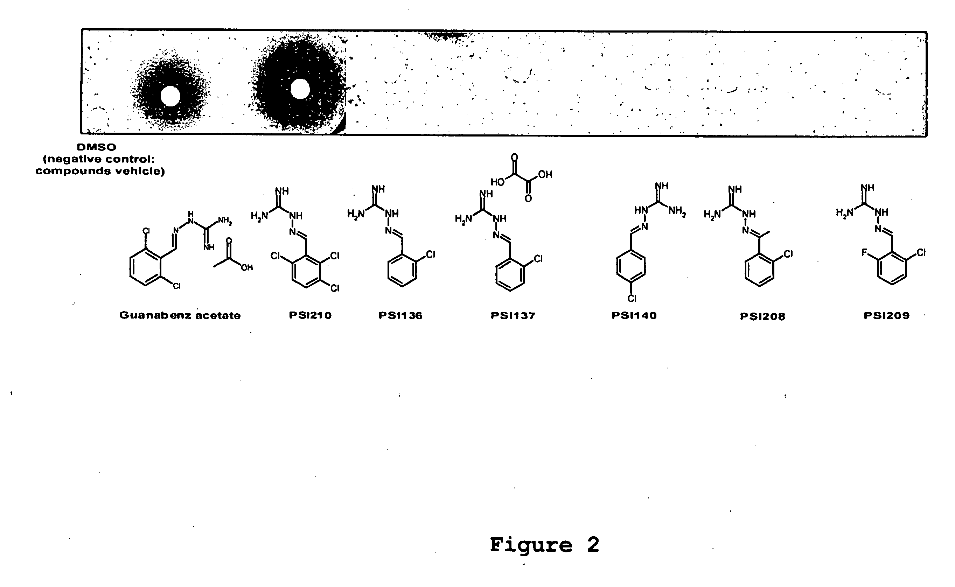

### Figure 2: Activity of chemical derivatives of Guanabenz

The same quantity of Guanabenz acetate and of six derivatives was spotted on the filters following the same protocol as the one described in figure 1. Note the lack of activity of five analogs and the strongest activity of the trichloro derivative (PSI210).

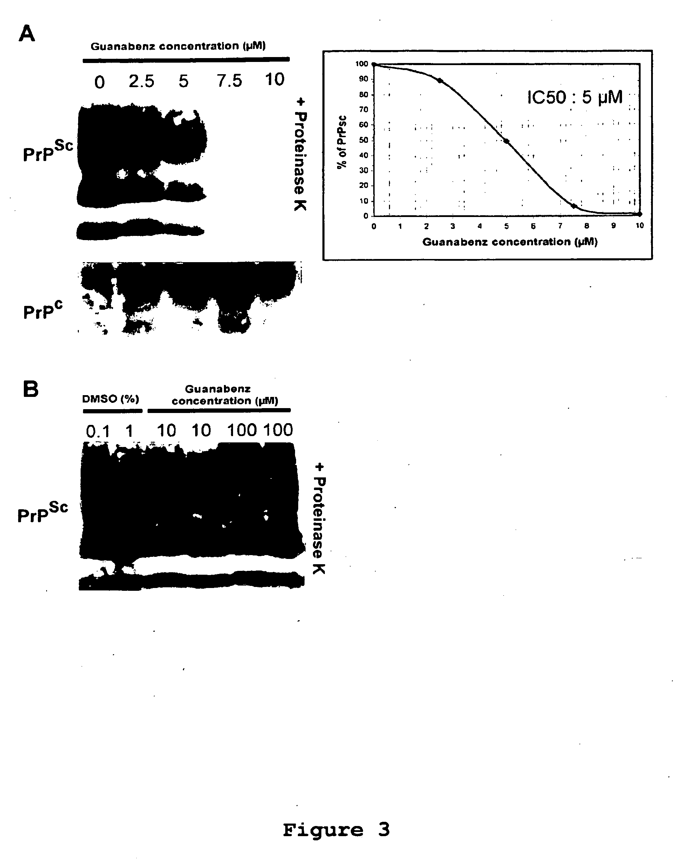

### Figure 3: Guanabenz efficiently promotes PrP^{Sc} clearance in an ex vivo murine cell-based assay

**A.** Scrapie-infected MovS cells were treated for 7 days with the indicated concentrations of Guanabenz acetate and then lysed. Cell lysates were then subjected to proteinase K digestion followed by Western blot analysis using an anti-PrP antibody (upper gel) or directly analyzed by Western blot analysis using an anti-PrP antibody before proteinase K treatment (lower gel). On the right a graph representing the quantity of remaining PrP^{Sc} as a function of Guanabenz acetate concentration is shown. Note that Guanabenz acetate was active against PrP^{Sc} in a dose-dependent manner (IC₅₀: 5 µM) and without any significant effect on PrP expression.
**B.** Cell lysates of scrapie-infected MovS cells were subjected directly to the indicated concentration of Guanabenz acetate or, as controls to the corresponding quantity of DMSO, the compound vehicle, and then subjected to proteinase K digestion followed by Western blotting analysis using an anti-PrP antibody. Note that Guanabenz, even at 100 µM (20 times the IC₅₀) was unable to resolubilize PrP^{Sc} indicating that it probably does not act directly on pre-existing prion aggregates.

### Figure 4:

**A.** Scrapie-infected mice expressing ovine PrP were treated by intraperitoneal injection once a week or not (Control) with Guanabenz acetate at 10 mg/kg. After 49 days of treatment the amount of PrP^{Sc} detectable in the spleen was determined using the proteinase K resistance assay as described in figure 3. Note that spleens of mice treated with Guanabenz acetate contain significantly less PrP^{Sc} than spleens of control, untreated mice.
**B.** Quantification of the Western blot showed above.

### Example 1: Isolation of Guanabenz as active against yeast prion

**Yeast strains and culture media.** Yeast strains used in this study were as follows. Strg6: *Mata, erg6::TRP1, adel-14, trpl-289, his3Δ200, ura3-52, leu2-3,112, [PSI⁺]* (Bach et al., 2003a) and SB34: *Mata, erg6::TRP1, dal5::ADE2, ade2-1, trp1-1, leu2-3,112, his3-11,15, ura2::HIS3,* [URE3] (Bach et al., 2003a).

Standard yeast growth conditions and genetic manipulations were as described (Guthrie and Fink, 1991).

**Result.** Using the two step yeast-based assay described previously (Bach et al., 2003a; Bach et al., 2002; Bach et al., 2003b), the inventors screened a library of compounds constituted of drugs which are either in phase II/III clinical trials or already marketed and used in clinic.

The Prestwick chemical library was chosen which is composed of 880 molecules. Among all these drugs, Guanabenz acetate was isolated as very active against [PSI⁺] prion (Figure 1 panel A and panel B, left). Guanabenz is an agonist of α2-adrenergic receptor used in the treatment of hypertension. Guanabenz acetate was then evaluated against [URE3], a second yeast prion and found to be also very active (Figure 1 panel B, right).

### Example 2: Activity of Guanabenz derivatives

**Result.** Six derivatives of Guanabenz (PSI136, PSI137, PSI140, PSI203, PSI209 and PSI210) were next tested among which four retain only one of chlorine substituents present in Guanabenz. All these four molecules turned out to be totally inactive against yeast [*PSI⁺*] prion (Figure 2) highlighting the importance of these two chlorines. This also confirmed the specificity of the screening method. Interestingly, PSI210 which contain an additional chlorine is more potent than Guanabenz itself, highlighting again the importance of the chlorine.

In addition the inventors also tested, Clonidine, a compound which is pharmacologically and chemically very close to Guanabenz and also used in clinic as an agonist of α2-adrenergic receptor for the treatment of hypertension. Clonidine did not exhibit any anti-prion activity (data not shown) indicating that the anti-prion activity of Guanabenz probably does not involve the same mechanism than the one related to its hypotensive action.

### Example 3: Guanabenz is able to efficiently promote PrP^{Sc} clearance in an ex vivo cell-based assay

**PrP^{Sc} inhibition assay in MovS cells.** Murine neuroglial MovS cells infected with ovine prions (Archer et al., 2004) were split and grown for 7 days in the presence of the indicated concentrations of drugs. Media and drugs were changed at half incubation. Cultures were then solubilized with detergent and analyzed by immunoblotting for the presence of normal or abnormal, resistant to PK digestion PrP, as described previously (Vilette et al., 2001).

**Result.** The activity of Guanabenz acetate was evaluated against mammalian prion using the above mentioned cell-based assay. This assay is based on a murine neuroglial cell line expressing ovine PrP gene under the control of its endogenous promoter (MovS cells). Cells were then infected by an homogenate prepared from the brains of transgenic mice infected with sheep prions. These scrapie-infected MovS cells could then grow, divide and propagate PrP^{Sc}. The state of PrP^{Sc} was monitored using a proteinase K sensitivity assay. Guanabenz acetate was found to be active against mammalian prion in this cell based-assay (Figure 3 panel A) . By testing different concentrations of Guanabenz acetate, a dose-dependent antiprion effect was observed which allow the inventors to determine an IC₅₀ of about 5µM for Guanabenz acetate (Figure 3, panel A, right). In the same experiment the level of PrP was followed to observe if Guanabenz acetate could have an effect on its expression in MovS cells (Figure 3 panel A, bottom). As the PrP level remained unchanged (as judged by Western blot analysis on the protein extracts before proteinase K treatment), the inventors conclude that Guanabenz acetate is active against PrP^{Sc} and does not act by decreasing the level of PrP expression which could indirectly have an effect on the level of PrP^{Sc}.

Protein lysates from infected MovS cells were also incubated for two hours with 10 or 100µM of Guanabenz acetate and then submitted to proteinase K assay in order to observe if this drug would be able to act direcly on pre-existing PrP^{Sc} aggregates by dissolving them. As shown in Figure 3, panel B, even with 100µM Guanabenz acetate (which represents 20 times the IC₅₀ value observed in cell culture) the level of PrP^{Sc} remained unchanged meaning that this molecule is not able to solubilize pre-existing PrP^{Sc} aggregates.

### Example 4: Guanabenz promotes PrP^{Sc} clearance in an in vivo murine model for prion-based disease

**PrP^{Sc} inhibition in transgenic mice.** Transgenic mice (tg338) expressing the ovine PrP and highly susceptible to sheep prion infection (Vilotte et al., 2001) were infected by intraperitoneal inoculation with 100 ul of 10% brain homogenate from transgenic mice terminally infected with sheep prions. Infected mice were then treated weekly by intraperitoneal infection of Guanabenz acetate (10 mg/kg). Levels of abnormal PrP in the spleen were determined as described previously (Beringue et al., 2000).

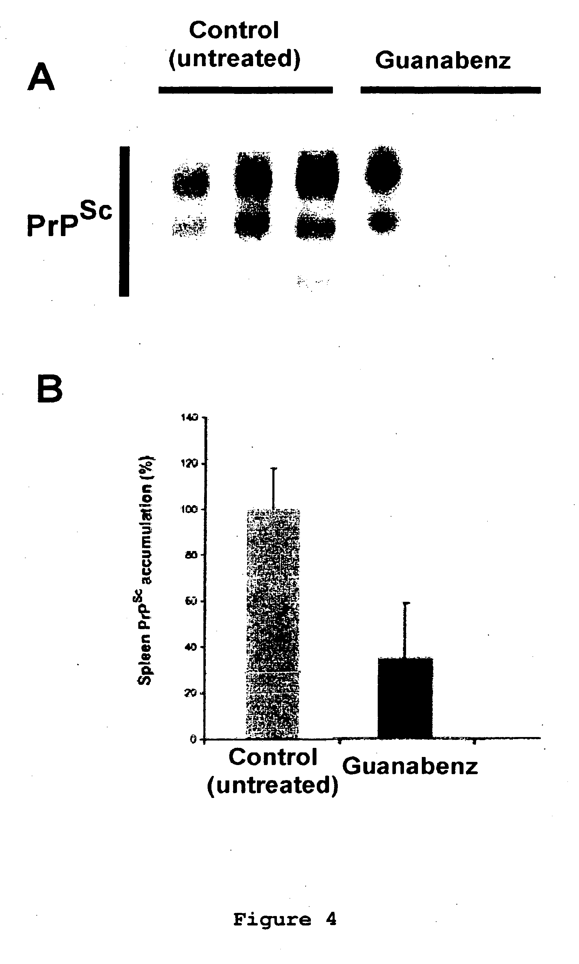

**Result.** The *in vivo* effect of Guanabenz acetate on mammalian prion was then evaluated using the above mentioned mouse model for prion-based disease. Mice expressing ovine PrP were scrapie-infected by intraperitoneal inoculation with the sheep scrapie agent and then treated once a week or not with Guanabenz acetate (10mg/kg). In this model, due to the progress of infection, the presence of PrP^{Sc} can be monitored in the spleen already a few weeks after infection reaching a plateau after 4 weeks. 49 days after infection, the level of PrP^{Sc} was determined in the spleen of untreated mice (Figure 4, panel A, left) and of mice treated with Guanabenz acetate (Figure 4, panel A, right). A significant decrease in the quantity of PrP^{Sc} was observed, indicating that Guanabenz acetate is able to decrease or slow down the infectious process. As a positive control, Dextran Sulfate 500 was used that was already known to have such an effect (Beringue et al., 2000).

### Example 5: Therapeutic composition comprising Guanabenz, for treating Creutzfeldt-Jakob's disease.

Composition of a tablet suitable for oral administration:
Guanabenz acetate
Lactose
Dicalcium phosphate
Corn starch
Colloidal silica
Povidone
Stearic acid
Soluble starch

Posology
4 mg of guanabenz acetate, twice a day.

### References

Archer, F., Bachelin, C., Andreoletti, O., Besnard, N., Perrot, G., Langevin, C., Le Dur, A., Vilette, D., Baron-Van Evercooren, A., Vilotte, J.L. and Laude, H. (2004) Cultured peripheral neuroglial cells are highly permissive to sheep prion infection. J Virol, 78, 482-490.
Bach, S., Talarek, N., Andrieu, T., Vierfond, J.M., Mettey, Y., Galons, H., Dormont, D., Meijer, L., Cullin, C. and Blondel, M. (2003a) Isolation of drugs active against mammalian prions using a yeast-based screening assay. Nat Biotechnol, 21, 1075-1081.
Bach, S., Talarek, N., Vierfond, J.-M., Mettey, Y., Cullin, C. and Blondel, M. (2002) Criblage de molecules à activité anti-prion : kits, méthodes et molécules criblées. *Demande de brevet français,* numéro d'enregistrement : 0213022.
Bach, S., Talarek, N., Vierfond, J.-M., Mettey, Y., Cullin, C. and Blondel, M. (2003b) Criblage de molecules à activité anti-prion : kits, méthodes et molécules criblées. *Demande de Brevet* international, PCT/FR03/03101.
Beringue, V., Adjou, K.T., Lamoury, F., Maignien, T., Deslys, J.P., Race, R. and Dormont, D. (2000) Opposite effects of dextran sulfate 500, the polyene antibiotic MS-8209, and Congo red on accumulation of the protease-resistant isoform of PrP in the spleens of mice inoculated intraperitoneally with the scrapie agent. J Virol, 74, 5432-5440.
Guthrie, C. and Fink, G.R. (1991) Guide to Yeast Genetics and Molecular Biology. Academic Press Inc., San Diego, California, U.S.A.
Vilette, D., Andreoletti, O., Archer, F., Madelaine, M.F., Vilotte, J.L., Lehmann, S. and Laude, H. (2001) Ex vivo propagation of infectious sheep scrapie agent in heterologous epithelial cells expressing ovine prion protein. Proc Natl Acad Sci U S A, 98, 4055-4059.
Vilotte, J.L., Soulier, S., Essalmani, R., Stinnakre, M.G., Vaiman, D., Lepourry, L., Da Silva, J.C., Besnard, N., Dawson, M., Buschmann, A., Groschup, M., Petit, S., Madelaine, M. F., Rakatobe, S., Le Dur, A., Vilette, D. and Laude, H. (2001) Markedly increased susceptibility to natural sheep scrapie of transgenic mice expressing ovine prp. J Virol, 75, 5977-5984.

## Claims

1. Use of the molecule of formula: 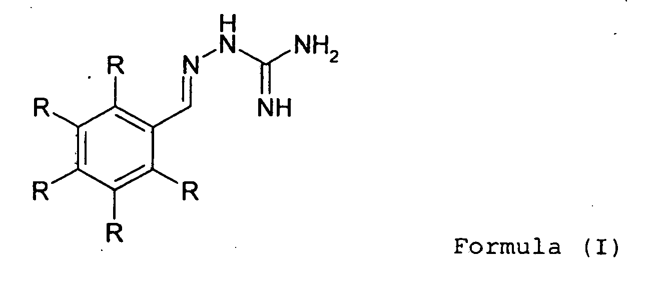 wherein R = H or Cl and the phenyl group is at least substituted twice, or a pharmaceutically acceptable salt thereof, for the manufacture of a medicament for treating prion-based diseases.

2. Use according to claim 1, wherein the molecule is of formula: 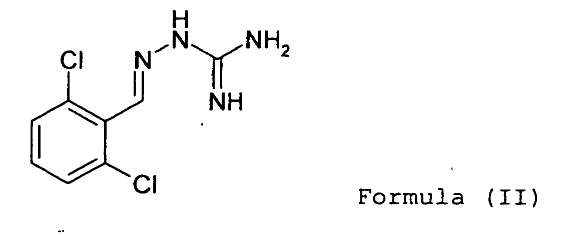 or a pharmaceutically acceptable salt thereof, for the manufacture of a medicament for treating prion-based diseases.

3. Use according to claim 2, wherein the molecule is the acetate salt of formula: 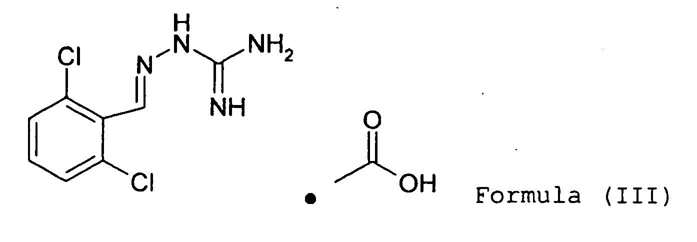 for the manufacture of a medicament for treating prion-based diseases.

4. Use according to claim 1, wherein the molecule is of formula: 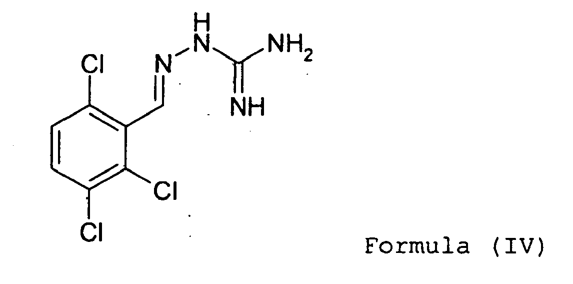 or a pharmaceutically acceptable salt thereof, for the manufacture of a medicament for treating prion-based diseases.

5. Use according to any of the preceding claims, wherein the prion-based disease is selecting in the group consisting of bovine spongiform encephalopathy, Creutzfeldt-Jakob disease, Gerstmann-Straussler-Scheinker syndrome, fatal familial insomia, kuru, scrapie, chronic wasting disease, feline spongiform encephalopathy and exotic ungulate encephalopathy.

6. Use according to claim 5, wherein the prion-based disease is selecting in the group consisting of bovine spongiform encephalopathy, Creutzfeldt-Jakob disease, kuru, scrapie.

## Patentansprüche

1. Verwendung des Moleküls der Formel 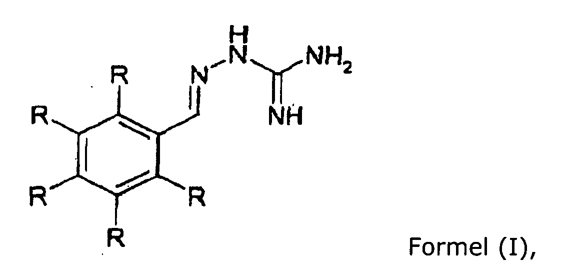 wobei R = H oder Cl ist und die Phenylgruppe wenigstens zweifach substituiert ist, oder eines pharmazeutisch annehmbaren Salzes davon zur Herstellung eines Medikaments zur Behandlung von Prionenkrankheiten.

2. Verwendung gemäß Anspruch 1, wobei das Molekül die Formel 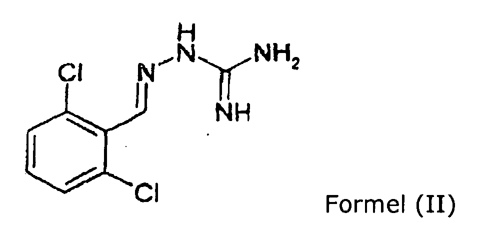 hat, oder eines pharmazeutisch annehmbaren Salzes davon zur Herstellung eines Medikaments zur Behandlung von Prionenkrankheiten.

3. Verwendung gemäß Anspruch 2, wobei es sich bei dem Molekül um das Acetatsalz der Formel 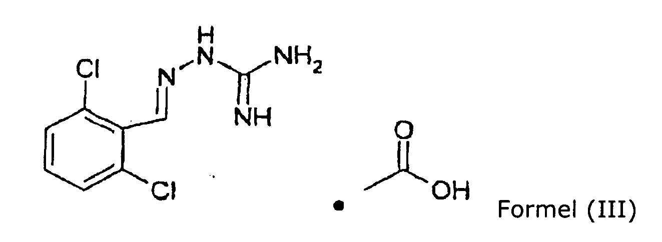 handelt, zur Herstellung eines Medikaments zur Behandlung von Prionenkrankheiten.

4. Verwendung gemäß Anspruch 1, wobei das Molekül die Formel 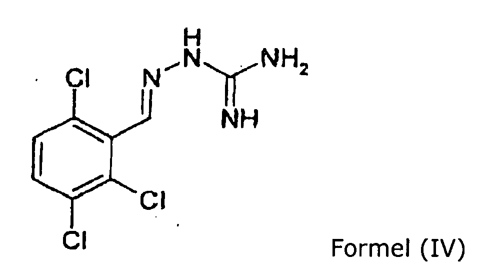 hat, oder eines pharmazeutisch annehmbaren Salzes davon zur Herstellung eines Medikaments zur Behandlung von Prionenkrankheiten.

5. Verwendung gemäß einem der vorstehenden Ansprüche, wobei die Prionenkrankheit aus der Gruppe ausgewählt ist, die aus boviner spongiformer Enzephalopathie, Creutzfeldt-Jakob-Krankheit, Gerstmann-Sträussler-Scheinker-Syndrom, tödlicher familiärer Schlaflosigkeit, Kuru, Scrapie, CWD (Chronic Wasting Disease), feliner spongiformer Enzephalopathie und EUE (Exotic Ungulate Encephalopathy) besteht.

6. Verwendung gemäß Anspruch 5, wobei die Prionenkrankheit aus der Gruppe ausgewählt ist, die aus boviner spongiformer Enzephalopathie, Creutzfeldt-Jakob-Krankheit, Kuru und Scrapie besteht.

## Revendications

1. Utilisation de la molécule de formulé : 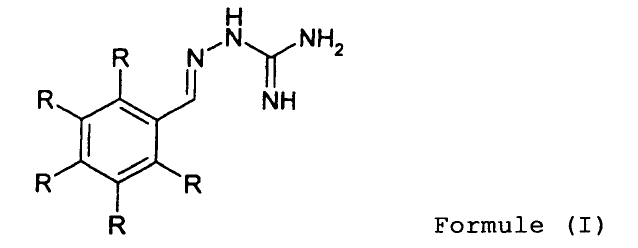 dans laquelle R = H ou Cl et le groupe phényle est au moins substitué deux fois, ou d'un sel pharmaceutiquement acceptable, pour la fabrication d'un médicament pour le traitement des maladies à prions.

2. Utilisation selon la revendication 1, dans laquelle la molécule est de formule : 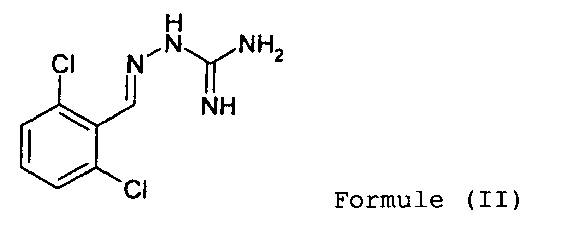 ou d'un sel pharmaceutiquement acceptable, pour la fabrication d'un médicament pour le traitement des maladies à prions.

3. Utilisation selon la revendication 2, dans laquelle la molécule est le sel acétate de formule : 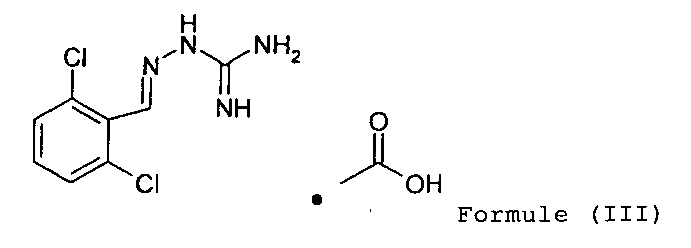 pour la fabrication d'un médicament pour le traitement des maladies à prions.

4. Utilisation selon la revendication 1, dans laquelle la molécule est de formule : 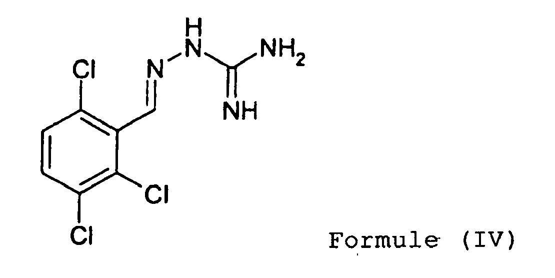 ou d'un sel pharmaceutiquement acceptable, pour la fabrication d'un médicament pour le traitement des maladies à prions.

5. Utilisation selon l'une quelconque des revendications précédentes, dans laquelle la maladie à prions est choisie dans le groupe formé par l'encéphalopathie spongiforme bovine, la maladie de Creutzfeldt-Jakob, le syndrome de Gerstmann-Sträussler-Scheinker, l'insomnie familiale fatale, le kuru, la tremblante du mouton, la maladie du dépérissement chronique des cervidés, l'encéphalopathie spongiforme féline et l'encéphalopathie des ongulés exotiques.

6. Utilisation selon la revendication 5, dans laquelle la maladie à prions est choisie dans le groupe formé par l'encéphalopathie spongiforme bovine, la maladie de Creutzfeldt-Jakob, le kuru, la tremblante du mouton.
